(19) Europäisches Patentamt
European Patent Office
Office européen des brevets

(11) Veröffentlichungsnummer: **0 013 056**
**B1**

(12) # EUROPÄISCHE PATENTSCHRIFT

(45) Veröffentlichungstag der Patentschrift: **10.02.82**

(51) Int. Cl.³: **A 61 K 9/02**, A 61 K 31/16

(21) Anmeldenummer: **79200784.1**

(22) Anmeldetag: **20.12.79**

(54) **Vaginales Kontrazeptivum.**

(30) Priorität: **22.12.78 CH 13146/78**

(43) Veröffentlichungstag der Anmeldung: **09.07.80 Patentblatt 80/14**

(45) Bekanntmachung des Hinweises auf die Patenterteilung: **10.02.82 Patentblatt 82/6**

(84) Benannte Vertragsstaaten: **AT BE DE FR GB IT LU NL SE**

(56) Entgegenhaltungen:
**FR-A-2 264 522**
**GB-A-823 025**
**NL-A-69 02076**
**NL-A-73 02521**
**US-A-2 623 840**
**US-A-3 876 757**
**CHEMINAL ABSTRACTS, Band 57, Nr. 4, 20. August 1962, Spalte 5274g, Columbus, Ohio, US, CLARE HARVEY: «Spermicidal activity of surface-active agents».**

(73) Patentinhaber: **Lücker, Peter Wolfgang, Prof. Dr., Am Rebstöckel 13, D-6719 Bobenheim am Berg (DE)**
Patentinhaber: **Wetzelsberger, Nikolaus, Dr., Dürerstrasse 12, D-6700 Ludwigshafen am Rhein (DE)**

(72) Erfinder: **Der Erfinder hat auf seine Nennung verzichtet**

(74) Vertreter: **Frossard, Michel, Dr. et al, A. Braun, Braun, Héritier, Eschmann AG, Patentanwälte Holbeinstrasse 36-38, CH-4051 Basel (CH)**

EP 0 013 056 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG.

# Vaginales Kontrazeptivum

Gegenstand der Erfindung ist ein neues, zur vaginalen Applikation bestimmtes Kontrazeptivum auf der Basis eines spermiziden Wirkstoffes, eines bei Wasseraufnahme Kohlendioxid entwikkelnden Gemisches eines Alkalimetallhydrogencarbonats und einer wasserlöslichen, festen, wasserfreien Mono- oder Dicarbonsäure mit 2 bis 6 Kohlenstoffatomen und eines bei Körpertemperatur schmelzenden Polyäthylenglykols oder Gemisches von Polyäthylenglykolen.

Seit etwa 15 Jahren finden sich im Handel oral wirkende Kontrazeptiva der Steroidreihe; sie bestehen meistens aus der Kombination einer gestagen- und einer östrogenwirkenden Verbindung. In bezug auf Sicherheit der Kontrazeption erfüllen diese Präparate den Zweck, schwankt doch ihr Pearlindex je nach Literaturangabe zwischen 0,0 und 2,3 bzw. zwischen 1,6 und 2,1 (J. Brotherton, Sex Hormone Pharmacology, S. 211–212, Academic Press, London 1976). Der Nachteil aller gestagenhaltigen Zubereitungen muss jedoch generell in dem massiven Eingriff in das hormonale System der Frau während einer Zeitspanne von möglicherweise 20 bis 30 Jahren gesehen werden, dessen volle Auswirkungen heute noch nicht überschaubar sind. Ausserdem werden orale Kontrazeptiva nicht von allen Frauen vertragen, da die Gestagenwirkung in manchen Fällen zu einer schwangerschaftsähnlichen Symptomatik führt, welche auch das beigegebene Östrogen nicht immer verhindern kann. Es bestehen ferner gewisse Kontraindikationen, u.a. der variköse Symptomenkomplex, eine Phlebitisvorgeschichte, Diabetes und Bluthochdruck. Aus diesen Gründen ist in letzter Zeit eine unverkennbare Abkehr von diesen Präparaten zu beobachten.

Andererseits sind bereits eine Vielzahl lokal anwendbarer Kontrazeptiva vorgeschlagen worden, welche im allgemeinen einen spermiziden Wirkstoff und ein Dispergiermittel enthalten. Unter den Wirkstoffen trifft man Verbindungen der verschiedensten chemischen Strukturen, von den metallorganischen Derivaten (z.B. Phenylquecksilber-nitrat und -acetat) über quaternäre Ammoniumsalze (z.B. Benzalkoniumchlorid, Benzethoniumchlorid, Cetylpyridiniumchlorid) bis zu Naturstoffen pflanzlicher Herkunft (z.B. Saponine) und Stoffe von rein synthetischer Art.

Die neuere und durchschlagende Entwicklung auf diesem Gebiet ist 1951 von F.V. Sander (US-Patent Nr. 2 541 103) eingeleitet worden, welcher durch Verwendung eines Alkylphenoxypolyäthoxyäthanols als Dispergiermittel eine deutliche Steigerung der spermiziden Wirkung erzielen konnte. Von den dort zur Verwendung vorgeschlagenen Verbindungen hat sich in der Folge das p-Nonylphenoxypolyäthoxyäthanol der Formel:

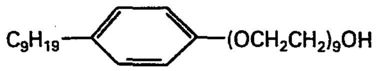

oder Nonoxynol 9 besonders durchgesetzt; es kommt heute, als alleiniger oder in Kombination mit einem anderen spermiziden Wirkstoff, in den meisten im Handel befindlichen lokalen Kontrazeptiva zur Anwendung. Eine bessere, einheitliche Verteilung des Wirkstoffes innerhalb der Scheide schliesslich soll der Zusatz eines einen Schaum erzeugenden Stoffgemisches, beispielsweise Natriumhydrogencarbonat und primäres Natriumphosphat (Brit. Patent Nr. 1 053 615), bewirken. Der Schaum kann gegebenenfalls durch Zumischung eines Schaumstabilisators, wie Polyvinylpyrrolidon oder Methylcellulose, oder er soll sogar durch Zugabe von Natriumlaurylsulfat (Deutsches Patent Nr. 2 213 604) über einen längeren Zeitraum erhalten bleiben. Eines der zur Zeit bekanntesten und meistverwendeten lokalen Kontrazeptiva weist die zuletzt erwähnte Ausgestaltung auf.

Dennoch scheint die Entwicklung noch nicht zu einem allseits oder voll befriedigenden Abschluss gekommen zu sein, sei es, dass bei der Anwendung gewisse Nebenerscheinungen, z.B. ein Austreten des Schaumes aus der Scheide, als störend empfunden werden, sei es vor allem, dass bei einigen Präparaten kein klares Bild in bezug auf Sicherheit der Kontrazeption herrscht oder dass sie nicht an jene der oral wirkenden Kontrazeptiva herankommt (H. Schmidt-Matthiesen, Gynäkologie und Geburtshilfe, 3. Auflage, Seite 143, F. K. Schattauer Verlag, Stuttgart-New York 1976).

Die mit den lokal anwendbaren Kontrazeptiva eingeschlagene Richtung verdiente aber zweifellos eine Fortführung der bisherigen Anstrengungen, weil dadurch im Gegensatz zu den Steroidpräparaten keine systemische Wirkung erzeugt, die Physiologie der Frau also nicht angetastet wird und keine Nebenwirkungen und Spätwirkungen zu erwarten sind und weil andererseits die Anwendung, mindestens bei sensiblen Menschen, als annehmbarer denn jene mechanischer Verhütungsmittel empfunden wird.

So wurde nun ein lokal anwendbares Kontrazeptivum gefunden, welches in bezug auf Bequemlichkeit der Anwendung und Atoxizität den bekanntesten vergleichbaren Präparaten nicht nachsteht, jene aber in bezug auf Sicherheit des herbeigeführten Schutzes deutlich übertreffen dürfte.

Das erfindungsgemässe Kontrazeptivum ist dadurch gekennzeichnet, dass es als spermiziden Wirkstoff ein N-(Hydroxyäthyl-polyoxyäthyl)-carbonsäureamid der Formel:

$$RCONH{-}(CH_2CH_2O)_n{-}H$$

in welcher R einen Alkyl- oder Alkenylrest mit 5 bis 19 Kohlenstoffatomen bedeutet und n einen Wert von 3 bis 20 hat, in zur sicheren Schwangerschaftsverhütung genügender Menge und das Kohlendioxid entwickelnde Stoffgemisch in stöchiometrischem Verhältnis der Komponenten und

in zur Erzeugung des Schaumes gerade ausreichender Menge enthält.

In der obigen Formel stellt die RCO-Gruppe den Acylrest einer aliphatischen, gesättigten oder ungesättigten Carbonsäure mit 6 bis 20 C-Atomen dar. Beispiele geeigneter Acylreste sind – im unteren Bereich der Definition von R – jene der Capronsäure, Önanthsäure, Caprylsäure, Pelargonsäure, Caprinsäure und Undecylsäure oder der Sorbinsäure, Citronellsäure und Undecylensäure. Bevorzugt wird die Verwendung eines Carbonsäureamids aus dem oberen Bereich, d.h. einer Verbindung der obigen Formel, in welcher R einen Alkyl- oder Alkenylrest mit 11 bis 19 Kohlenstoffatomen bedeutet bzw. die RCO-Gruppe dem Acylrest einer aliphatischen Carbonsäure mit 12 bis 20 C-Atomen entspricht. Beispiele solcher Acylreste sind jene der Laurinsäure, Myristinsäure, Palmitinsäure, Margarinsäure, Stearinsäure und Arachinsäure oder der Palmitoleinsäure, Ölsäure, Linolsäure, Linolensäure und Eläostearinsäure. Es können auch Gemische zweier oder mehrerer solcher Carbonsäureamide verwendet werden, wie sie durch Herstellung aus verschiedenen Naturprodukten, insbesondere aus Pflanzenölen wie Kokosnussöl und Erdnussöl erhalten werden.

In der obigen Formel hat n vorzugsweise einen Mittelwert von 4 bis 12. Zusammen mit der oben erwähnten, bevorzugten Bedeutung von R bzw. RCO– ergeben sich daraus als Beispiele von besonders bevorzugten Carbonsäureamiden solche der Formel, in welcher RCO– den Oleoylrest und n die Zahl 8 bedeuten oder RCO– einen Lauroyl-, Myristoyl-, Palmitoyl-, Stearoyl- oder Arachinoylrest oder ein Gemisch zweier oder mehrerer dieser Reste, wie es bei der sauren Hydrolyse von Kokosnussöl entsteht, bedeutet und n einen Mittelwert von 6 hat.

Als Kohlendioxid bildende Komponente des Schaumgenerators seien das Kalium- und das Natriumhydrogencarbonat genannt; bevorzugt wird letzteres. Als Mono- oder Dicarbonsäure eignen sich insbesondere Hydroxicarbonsäuren, wie Glykolsäure, l-Milchsäure, Äpfelsäure oder Weinsäure, und Aminodicarbonsäuren, wie Glutaminsäure, Hydroxiglutaminsäure oder α-Aminoadipinsäure; bevorzugt wird Weinsäure. Im Gemisch von Natriumhydrogencarbonat und Weinsäure finden sich die beiden Komponenten in einem molaren Verhältnis von 2 zu 1.

Als bei Körpertemperatur schmelzendes Polyäthylenglykol oder Polyäthylenglykolgemisch eignet sich z.B. ein Gemisch der Polyäthylenglykole 400 und 1500 in einem Gewichtsverhältnis von ca. 0,317:1,906, oder ein Gemisch der Polyäthylenglykole 1000 und 1350 in einem Gewichtsverhältnis von 1:1, oder ein Gemisch der Polyäthylenglykole 400, 1540 und 1600 in einem Gewichtsverhältnis von ca. 12,7:19,0:31,7.

In dem erfindungsgemässen Kontrazeptivum soll der spermizide Wirkstoff unter Berücksichtigung einer einheitlichen Verteilung innerhalb der Scheide in zur sicheren Schwangerschaftsverhütung genügender, das Gemisch von Alkalimetallhydrogencarbonat und Mono- oder Dicarbonsäure in zur Erzeugung des Schaums gerade ausreichender Menge vorliegen. Eine bevorzugte Ausführungsform des Kontrazeptivums ist diesbezüglich insofern ausgewogen, als sie aus ca. 2 bis 10 Gew.-% N-(Hydroxyäthyl-polyoxyäthyl)-carbonsäureamid, ca. 8 bis 25 Gew.-% Weinsäure-Natriumhydrogencarbonat-Gemisch (im molaren Verhältnis 1:2) und ca. 65 bis 90 Gew.-% Polyäthylenglykol oder Polyäthylenglykolgemisch besteht.

Die Herstellung des Kontrazeptivums erfolgt dadurch, dass man das Polyäthylenglykol oder Polyäthylenglykolgemisch durch leichtes Erwärmen zum Schmelzen bringt und der Schmelze unter Rühren die berechnete Menge pulverisierte Mono- oder Dicarbonsäure, pulverisiertes Alkalimetallhydrogencarbonat und N-(Hydroxyäthylpolyoxyäthyl)-carbonsäureamid zugibt. Nach vollständiger Homogenisierung kann die Masse unter weiterem Rühren in Suppositorienformen oder Ovulaformen gegossen werden.

Die Carbonsäureamide der obigen Formel sind bekannt und einige werden auch praktisch verwendet. Beispielsweise wird das im nachfolgenden Beispiel 1 verwendete Carbonsäureamidgemisch insbesondere als Fliessmittel in Kinderpudern gebraucht, was seine ausgezeichnete Verträglichkeit beweist.

Um ein genaues Bild über die Toxizität der neuen Wirkstoffe zu erhalten, ist das Carbonsäureamid von Beispiel 1 (aus Kokosnussöl; R = Gemisch von $C_{11}H_{23}$, $C_{13}H_{27}$, $C_{15}H_{31}$, $C_{17}H_{35}$ und $C_{19}H_{39}$, Mittelwert von n = 6) einer Prüfung auf akute Toxizität – $LD_{50}$ in mg/kg – bei der Ratte und dem Kaninchen nach einmaliger peroraler Verabreichung (unverdünnt) unterzogen worden.

| Zeitpunkt | $LD_{50}$ Ratte | $LD_{50}$ Kaninchen |
|---|---|---|
| nach 24 Stunden | 4'404 | 2'049 |
| nach 14 Tagen | 3'684 | ca. 2'000 |

Unter den beschriebenen Versuchsbedingungen wurde festgestellt, dass der Wirkstoff von Beispiel 1 in der geprüften Dosierung beim Kaninchen eine leichte Aktivitätsverminderung mit Apathie hervorrief. Die Symptome hielten zum Teil über 24 Stunden an; danach zeigten die überlebenden Tiere wieder ein normales Verhalten. Die gestorbenen Tiere zeigten bei der Sektion leichte Hämorrhagien der Magenschleimhaut, die wahrscheinlich auf den pH-Wert von 8,28 zurückzuführen sind. Die beim Versuchsende, d.h. nach einer Beobachtungsdauer von 14 Tagen, getöteten Tiere wiesen bei der Sektion keine makroskopischen Organveränderungen auf. Bei der Ratte waren die motorischen Symptome, wie Aktivitätsverminderung und Haltungsanomalien, stärker ausgeprägt, die Erholung und die Befunde bei der Sektion waren jedoch jenen beim Kaninchen weitgehend ähnlich.

Abgesehen von der unbedeutenden Toxizität der erfindungsgemäss verwendeten Wirkstoffe

bestehen deren Spaltprodukte aus Fettsäuren, gleich jenen, welche im Organismus aus den Fetten und Ölen der Nahrung entstehen. Falls infolge besonderer Absorptionsverhältnisse in der Scheide der spermizide Wirkstoff vom Organismus teilweise aufgenommen werden sollte, sind also auch von den Spaltprodukten keine toxischen Wirkungen zu befürchten, was z.B. mit dem aus Nonoxynol 9 möglicherweise entstehenden p-Nonylphenol nicht gleicherweise erwartet werden kann.

Es wurde dann die Schaumentwicklung bei dem neuen Kontrazeptivum untersucht und mit jener eines vergleichbaren bekannten Präparates (Deutsches Patent Nr. 2 213 604) verglichen. Dazu wird ein Reagenzglas mit Volumengraduierung und Magnetrührer im Wasserbad bei 37 °C gehalten, mit 1 ml Wasser versehen und darin ein Ovulum oder Suppositorium gegeben; der Versuch wurde mit dem Suppositorium gemäss Beispiel 1 durchgeführt. Das Volumen des sich bildenden Schaumes wird in Abhängigkeit der Zeit gemessen. Wie folgende Tabelle zeigt, erreicht das erfindungsgemässe Kontrazeptivum das Maximum seiner Schaumentwicklung bereits nach 7,5 Minuten und es weist eine kürzere Zerfallszeit auf als das Vergleichspräparat, dessen Schaumentwicklung auch nach 20 Minuten noch nicht abgeschlossen ist.

Tabelle 1: Schaumvolumen in ml

| Zeit in Min. | Vergleichspräparat | Beispiel 1 |
|---|---|---|
| 1 | 2,8 | 5,5 |
| 2 | 4,0 | 6,2 |
| 3 | 4,8 | 8,5 |
| 5 | 5,5 | 10,2 |
| 7,5 | 6,8 | 11,5 |
| 10 | 9,2 | 10,5 |
| 15 | 12,0 | 6,8 |
| 20 | 14,5 | 5,8 |

Bei dem Suppositorium von Beispiel 1 fällt also der Schaum zusammen, wenn er seine Funktion, nämlich eine einheitliche Verteilung des spermiziden Wirkstoffs innerhalb der Scheide, erfüllt hat. Ein Austreten des Schaumes aus der Scheide, wie es bei dem herkömmlichen Präparat öfters als belästigend empfunden wird, dürfte daher kaum mehr erfolgen.

Lokale Kontrazeptiva enthalten als Wirkstoff Substanzen, die die Bewegung der Spermien einschränken oder vollkommen hemmen. Die Motilität der Spermien ist das einzige direkt erfassbare Kriterium deren Vitalität. Die Güte eines Kontrazeptivums wird naturgemäss davon abhängen, wie schnell die Immobilisation nach Kontakt des Spermas mit dem Wirkstoff eintritt. Von einem sicher wirkenden Kontrazeptivum sollte erwartet werden, dass die Immotilität innerhalb einer Minute nach Berührung vollständig erreicht ist.

Im folgenden wird über einen in einer Klinik durchgeführten Spermien-Immobilisationstest berichtet, bei welchem das Präparat gemäss Beispiel 1 und ein vergleichbares, Nonoxynol 9 enthaltendes Präparat untersucht wurden. Es stand frisches Sperma von 5 freiwilligen Spendern mit normalen Spermiogrammen zur Verfügung. Das Sperma wurde bis zur Beendigung der Verflüssigungsphase bei 37 °C in einer feuchten Kammer aufbewahrt. Die Präparate wurden bei 37 °C gelöst; das Präparat von Beispiel 1 war in weniger als 10 Minuten ohne Schütteln vollständig gelöst. Von den Lösungen wurden zwei Verdünnungen hergestellt:

Verdünnung I: 1 Ovulum in 50 ml physiologischer Kochsalzlösung,

Verdünnung II: Lösung I und physiologische Kochsalzlösung in einem Volumenverhältnis von 1:5.

0,1 ml des Spermas wurden zusammen mit 0,5 ml der Verdünnung I bzw. II auf den Objektträger eines Lichtmikroskops mit einer Pipette gegeben. Gleichzeitig wurde eine Stoppuhr eingeschaltet. Es wurde sodann in mehreren Feldern die Motilität der Spermien kontrolliert und so die Zeit bis zur vollständigen Immobilisation bestimmt. Die Messungen wurden jeweils in 2facher oder 3facher Bestimmung mit dem Sperma der Spender unabhängig voneinander durchgeführt. Da die Testergebnisse nach 150 Sekunden ungenau werden, wurde der Test jeweils zu dieser Zeit abgebrochen.

Die Einzelwerte des Immobilisationstests sind in Tabelle 2 dargestellt, ebenso die statistische Auswertung. Die Untersuchung ergab eindeutig, dass das neue Kontrazeptivum, enthaltend den Wirkstoff gemäss obiger Formel, in seiner spermienimmobilisierenden Wirkung dem Vergleichspräparat überlegen ist. Diese Aussage gilt für Verdünnung I und für Verdünnung II. Die Ergebnisse sind signifikant. Das Kontrazeptivum kann somit im in-vitro-Test als sicher spermienimmobilisierend bezeichnet werden.

Die erwähnten Ergebnisse sind durch eine erweiterte Untersuchung unter den oben geschilderten Bedingungen bestätigt worden. Die Proben wurden vom Laborpersonal blind (d.h. ohne Kenntnis der Zusammensetzung des verwendeten Präparates) gemessen; die Messungen wurden jeweils, ausser für das Präparat von Beispiel 1, in einfacher Bestimmung durchgeführt. In der Regel wurde der Test nach einer Minute abgebrochen, weil von einem sicher wirkenden Kontrazeptivum die Immobilisation in dieser Zeitspanne erwartet werden soll. Tabelle 3 fasst die Ergebnisse zusammen.

Tabelle 2:

| Immobilisationen in Sekunden Präparat | Spender 1 | Spender 2 | Spender 3 | Spender 4 | Spender 5 |
|---|---|---|---|---|---|
| Verdünnung I | | | | | |
| Vergleichs- | 79 | 150 | 58 | 120 | 70 |
| präparat | 150 | 82 | 55 | 75 | 40 |
| | 150 | 150 | | | |
| Beispiel 1 | 9 | 31 | 29 | 20 | 22 |
| | 18 | 33 | 30 | 22 | 18 |
| | 35 | 0 | | | |
| Verdünnung II | | | | | |
| Vergleichs- | 150 | 150 | 150 | 150 | 150 |
| präparat | 150 | 150 | 150 | 150 | 150 |
| | 150 | 150 | | | |
| Beispiel 1 | 44 | 24 | 38 | 32 | 20 |
| | 53 | 45 | 45 | 30 | 30 |
| | 27 | 72 | | | |

t-Test

| | | | |
|---|---|---|---|
| Verdünnung I: | $\bar{d}$= 75.0 | SD=45.09 | |
| | t= 5.84 | df=11 | p<0.001 |
| Verdünnung II: | $\bar{d}$=111.67 | SD=14.52 | |
| | t= 26.64 | df=11 | p<<0.002 |

Tabelle 3: Immobilisationszeiten in Sekunden

| Präparat | Spender 1 | | Spender 2 | | Spender 3 | |
|---|---|---|---|---|---|---|
| | Verdünnung I | Verdünnung II | Verdünnung I | Verdünnung II | Verdünnung I | Verdünnung II |
| physiologische Kochsalzlösung | – | – | – | – | > 10 Min. | > 10 Min. |
| Grundlage von Beispiel 1, ohne Wirkstoff | >60 | – | >60 | – | >180 | 155 |
| Vergleichspräparat | >60 | – | >60 | – | 133 | 105 |
| Beispiel 1 | <10 | 20 | <10 | 40 | < 20 | < 20 |
| | <10 | 60 | <10 | >60 | – | – |
| Beispiel 4 | 37 | >60 | 15 | 60 | – | – |
| Beispiel 5 | 20 | >60 | 30 | 60 | – | – |

Beispiel 1

317 g Polyäthylenglykol 400 und 1906 g Polyäthylenglykol 1500 werden unter wasserfreien Bedingungen bei 42 °C miteinander gemischt und geschmolzen und die Schmelze wird zur Homogenisierung während 15 Minuten gerührt. Sodann werden bei derselben Temperatur und unter fortwährendem Rühren 151 g pulverisierte Weinsäure, 156 g pulverisiertes Natriumhydrogencarbonat und 150 g Carbonsäureamid aus Kokosnussöl, entsprechend der zuvor gegebenen Formel mit RCO– = Gemisch von Lauroyl-, Myristoyl-, Palmitoyl-, Stearoyl- und Arachinoylresten und n (Mittelwert) = 6 [Handelsprodukt Steinapal C6 der Firma REWO Chemische Werke GmbH, D-6497 Steinau 1], zugegeben. Die Masse wird während weiteren 15 Minuten gerührt und unter ständigem Rühren in Suppositorienformen gegossen. Es werden 1000 Suppositorien mit einem durchschnittlichen Gewicht von 2,68 g erhalten.

Ein Suppositorium hat die folgende Zusammensetzung:

| | |
|---|---|
| spermizider Wirkstoff | 0,150 (= 5,59 Gew.-%) |
| Weinsäure | 0,151 |
| Natriumhydrogencarbonat | 0,156 |
| Polyäthylenglykol 400 | 0,317 |
| Polyäthylenglykol 1500 | 1,906 |

Wird ein Suppositorium in 20 ml Wasser bei 37 °C unter schwachem Rühren (Magnetrührer, ca. 100 Umdrehungen/Minute) gegeben, so soll es sich in weniger als 10 Minuten vollständig auflösen. Das oben erhaltene Suppositorium erfüllt diese Anforderung.

Beispiel 2

Es werden 317 g Polyäthylenglykol 400 und 1906 g Polyäthylenglykol 1500 bei 42 °C miteinander gemischt und geschmolzen und die Schmelze wird durch 15 Minuten Rühren homogenisiert. Danach werden unter ständigem Rühren und bei derselben Temperatur 151 g pulverisierte Weinsäure, 156 g pulverisiertes Natriumhydrogencarbonat und 150 g N-(Hydroxyäthyl-polyoxyäthyl)-ölsäureamid, entsprechend der zuvor gegebenen Formel mit RCO– = Oleoyl und n (Mittelwert) = 8 [Handelsprodukt Steinapal 08 der Firma REWO Chemische Werke GmbH, D-6497 Steinau 1], zugegeben. Es wird durch Rühren weitere 15 Minuten homogenisiert und die Schmelze unter ständigem Rühren in Ovulaformen gegossen.

Beispiel 3

Es werden 1111,5 g Polyäthylenglykol 1000 und 1111,5 g Polyäthylenglykol 1350 bei 42 °C miteinander gemischt und geschmolzen und die Schmelze wird bei derselben Temperatur während 15 Minuten gerührt. Sodann werden bei dieser Temperatur und unter ständigem Rühren 151 g pulverisierte Weinsäure, 156 g pulverisiertes Natriumhydrogencarbonat und 150 g Cocosamidpolyglycoläther [Handelsprodukt Steinapal C6, spez. Gew. 1,0243; siehe Beispiel 1], zugegeben. Die weitere Bearbeitung wird wie in Beispielen 1 und 2 durchgeführt.

Beispiel 4

Man verfährt nach der in Beispiel 1 beschriebenen Methode, jedoch unter Verwendung von 150 g N-(Hydroxyäthyl-polyoxyäthyl)-laurinsäureamid, entsprechend der zuvor gegebenen Formel mit RCO– = Lauroyl und n (Mittelwert) = 9, als spermizider Wirkstoff. Die Herstellung des Wirkstoffes erfolgte nach Schönfeldt, Grenzflächenaktive Äthylenoxidaddukte, Wissenschaftliche Verlagsgesellschaft, Stuttgart 1976, Seiten 67–69.

Beispiel 5

Man verfährt wie im Beispiel 2 unter Verwendung eines N-(Hydroxyäthyl-polyoxyäthyl)-ölsäureamids mit 9 Äthoxygruppen; obige Formel: RCO– = Oleoyl, n (Mittelwert) = 9 [Handelsprodukt Steinapal 08, REWO Chemische Werke GmbH].

Beispiel 6

Man arbeitet wie in den Beispielen 1 bis 3 beschrieben, verwendet aber 150 g N-(Hydroxyäthyl-polyoxyäthyl)-stearinsäureamid als spermizider Wirkstoff. In der gegebenen Formel bedeutet RCO– Stearoyl, n (Mittelwert) = 20. Die Herstellung des Wirkstoffs erfolgte nach derselben Literaturstelle wie in Beispiel 4.

**Patentansprüche**

1. Kontrazeptivum zur vaginalen Applikation auf der Basis eines spermiziden Wirkstoffes, eines bei Wasseraufnahme Kohlendioxid entwickelnden Gemisches eines Alkalimetallhydrogencarbonats und einer wasserlöslichen, festen, wasserfreien Mono- oder Dicarbonsäure mit 2 bis 6 Kohlenstoffatomen und eines bei Körpertemperatur schmelzenden Polyäthylenglykols oder Gemisches von Polyäthylenglykolen, dadurch gekennzeichnet, dass es als spermiziden Wirkstoff ein N-(Hydroxyäthyl-polyoxyäthyl)-carbonsäureamid der Formel:

$$RCONH-(CH_2CH_2O)_n-H$$

in welcher R einen Alkyl- oder Alkenylrest mit 5 bis 19 Kohlenstoffatomen bedeutet und n einen Wert von 3 bis 20 hat, in zur sicheren Schwangerschaftsverhütung genügender Menge und das Kohlendioxid entwickelnde Stoffgemisch in stöchiometrischem Verhältnis der Komponenten und in zur Erzeugung des Schaumes gerade ausreichender Menge enthält.

2. Kontrazeptivum nach Anspruch 1, dadurch gekennzeichnet, dass in obiger Formel R einen Alkyl- oder Alkenylrest mit 11 bis 19 Kohlenstoffatomen bedeutet.

3. Kontrazeptivum nach Anspruch 1 oder 2, dadurch gekennzeichnet, dass in obiger Formel n einen Mittelwert von 4 bis 12 hat.

4. Kontrazeptivum nach Anspruch 2 oder 3, dadurch gekennzeichnet, dass in obiger Formel die Gruppe RCO– den Lauroyl-, Myristoyl-, Palmitoyl-, Stearoyl-, Arachinoyl- oder Oleoylrest oder ein Gemisch zweier oder mehrerer derselben bedeutet.

5. Kontrazeptivum nach Anspruch 4, dadurch gekennzeichnet, dass die RCO-Gruppe dem Carbonsäuregemisch des sauren Hydrolysates von Kokosnussöl entspricht und n einen Mittelwert von 6 hat.

6. Kontrazeptivum nach Anspruch 4, dadurch gekennzeichnet, dass die RCO-Gruppe den Oleoylrest bedeutet und n einen Mittelwert von 8 hat.

7. Kontrazeptivum nach Anspruch 1, dadurch gekennzeichnet, dass die Mono- oder Dicarbonsäure aus einer Hydroxicarbonsäure, wie Glykolsäure, l-Milchsäure, Äpfelsäure oder Weinsäure, oder einer Aminodicarbonsäure, wie Glutaminsäure, Hydroxiglutaminsäure oder α-Aminoadipinsäure, besteht.

8. Kontrazeptivum nach Anspruch 1, dadurch gekennzeichnet, dass das bei Wasseraufnahme Kohlendioxid entwickelnde Stoffgemisch aus Natriumhydrogencarbonat und Weinsäure in einem Molverhältnis von 2:1 besteht.

9. Kontrazeptivum nach Anspruch 8, dadurch gekennzeichnet, dass das N-(Hydroxyäthyl-polyoxyäthyl)-carbonsäureamid in einer Gewichtsmenge von 2 bis 10%, Natriumhydrogencarbonat und Weinsäure in einem Molverhältnis von 2 zu 1 und einer Gewichtsmenge von 8 bis 25% und das Polyäthylenglykol oder Polyäthylenglykolgemisch in einer Gewichtsmenge von 65 bis 90% vorliegen.

10. Verwendung des Kontrazeptivums nach Anspruch 1 in Form eines Suppositoriums oder Ovulums zur Schwangerschaftsverhütung.

**Revendications**

1. Contraceptif pour application vaginale, à base d'une substance spermatocide, d'un mélange d'un hydrogénocarbonate de métal alcalin et d'un acide mono- ou dicarboxylique comportant de 2 à 6 atomes de carbone, soluble dans l'eau, solide et anhydre, mélange dégageant de l'anhydride carbonique par absorption d'eau, et d'un polyéthylène glycol ou d'un mélange de polyéthylène glycols fondant à la température du corps, caractérisé en ce qu'il contient comme substance spermatocide un N-(hydroxyéthyl polyoxyéthyl)-amide d'acide carboxylique de formule:

$$RCONH-(CH_2CH_2O)_n-H$$

dans laquelle R représente un radical alkyle ou alcényle comportant de 5 à 19 atomes de carbone et n a une valeur allant de 3 à 20, en quantité suffisante pour une prévention certaine de la grossesse, et qu'il contient le mélange dégageant de l'anhydride carbonique en proportion stoechiométrique des composants et en quantité juste suffisante pour former de la mousse.

2. Contraceptif selon la revendication 1, caractérisé en ce que R, dans la formule ci-dessus, représente un radical alkyle ou alcényle comportant de 11 à 19 atomes de carbone.

3. Contraceptif selon la revendication 1 ou 2, caractérisé en ce que n, dans la formule ci-dessus, a une valeur moyenne de 4 à 12.

4. Contraceptif selon la revendication 2 ou 3, caractérisé en ce que le groupe RCO–, dans la formule ci-dessus, représente le radical lauryle, myristyle, palmityle, stéaryle, arachinyle ou oléyle ou un mélange de deux ou plusieurs desdits radicaux.

5. Contraceptif selon la revendication 4, caractérisé en ce que le groupe RCO– correspond au mélange d'acides carboxyliques issu de l'hydrolyse acide de l'huile de noix de coco et que n a une valeur moyenne de 6.

6. Contraceptif selon la revendication 4, caractérisé en ce que le groupe RCO– représente le radical oléyle et que n a une valeur moyenne de 8.

7. Contraceptif selon la revendication 1, caractérisé en ce que l'acide mono- ou dicarboxylique est un acide hydroxy carboxylique, tel que l'acide glycolique, l-lactique, malique ou tartrique, ou un acide amino dicarboxylique, tel que l'acide glutamique, l'acide hydroxy glutamique ou l'acide α-amino adipique.

8. Contraceptif selon la revendication 1, caractérisé en ce que le mélange de substances dégageant de l'anhydride carbonique par absorption d'eau est un mélange d'hydrogénocarbonate de sodium et d'acide tartrique en un rapport moléculaire de 2 à 1.

9. Contraceptif selon la revendication 8, caractérisé en ce que le N-(hydroxyéthyl polyoxyéthyl)-amide d'acide carboxylique est présent à raison de 2 à 10% en poids, l'hydrogénocarbonate de sodium et l'acide tartrique sont présents en un rapport moléculaire de 2 à 1 et à raison de 8 à 25% en poids et le polyéthylène glycol ou le mélange de polyéthylène glycols est présent à raison de 65 à 90% en poids.

10. Utilisation du contraceptif selon la revendication 1, sous forme d'un suppositoire ou d'un ovule, pour la prévention de la grossesse.

**Claims**

1) Contraceptive for vaginal application, based on a spermicidal active ingredient, a mixture, which on absorption of water evolves carbon dioxide, of an alkali metal bicarbonate and a water-soluble, solid, anhydrous monocarboxylic acid or dicarboxylic acid with 2 to 6 carbon atoms, and a polyethylene glycol, or mixture of polyethylene glycols, which melts at body temperature, characterised in that it contains, as the spermicidal active ingredient, an N-(hydroxyethyl-polyoxyethyl)-carboxylic acid amide of the formula:

$$RCOHN-(CH_2CH_2O)_n-H$$

in which R denotes an alkyl or alkenyl radical with 5 to 19 carbon atoms and n has a value of 3 to 20, in an amount sufficient for reliable prevention of pregnancy, and that it contains the mixture of substances which evolve carbon dioxide in the stoichiometric ratio of the components and in an amount which just suffices to generate the foam.

2) Contraceptive according to Claim 1, characterised in that, in the above formula, R denotes an alkyl or alkenyl radical with 11 to 19 carbon atoms.

3) Contraceptive according to Claim 1 or 2, characterised in that in the above formula n has a mean value of 4 to 12.

4) Contraceptive according to Claim 2 or 3, characterised in that in the above formula the group RCO– denotes the lauroyl, myristoyl, palmitoyl, stearoyl, arachinoyl or oleoyl radical or a mixture of two or more of these.

5) Contraceptive according to Claim 4, characterised in that the RCO– group corresponds to the carboxylic acid mixture of the acid hydrolysate of coconut oil and that n has a mean value of 6.

6) Contraceptive according to Claim 4, characterised in that the RCO– group denotes the oleoyl radical and n has a mean value of 8.

7) Contraceptive according to Claim 1, characterised in that the monocarboxylic acid or dicarboxylic acid consists of a hydroxycarboxylic acid, such as glycolic acid, l-lactic acid, malic acid or tartaric acid, or of an aminodicarboxylic acid, such as glutamic acid, hydroxyglutamic acid or α-aminoadipic acid.

8) Contraceptive according to Claim 1, characterised in that the mixture which on absorption of water evolves carbon dioxide consists of sodium bicarbonate and tartaric acid in a molar ratio of 2:1.

9) Contraceptive according to Claim 8, characterised in that the N-(hydroxyethyl-polyoxyethyl)-carboxylic acid amide is present in a content of from 2 to 10% by weight, the sodium bicarbonate and tartaric acid are present in a molar ratio of 2:1 and a content of from 8 to 25% by weight and the polyethylene glycol or polyethylene glycol mixture is present in a content of from 65 to 90% by weight.

10) Use of the contraceptive according to Claim 1, in the form of a suppository or ovule, for the prevention of pregnancy.